# EUROPÄISCHE PATENTSCHRIFT

(11) **EP 1 223 874 B1**
(45) Veröffentlichungstag und Bekanntmachung des Hinweises auf die Patenterteilung: **28.12.2005**
(21) Anmeldenummer: 00969554.5
(22) Anmeldetag: 26.10.2000
(51) Int. Cl.: A61B 17/68

(54) **CHIRURGISCHES VERBINDUNGSELEMENT ZUR FIXIERUNG BENACHBART ANGEORDNETER KNOCHENPLATTEN**
SURGICAL CONNECTING ELEMENT FOR FIXING ADJACENT BONE PLATES
ELEMENT DE LIAISON CHIRURGICAL SERVANT A BLOQUER DES PLAQUES POUR OSTEOSYNTHESE ADJACENTES

(30) Priorität: 30.10.1999 DE 19952359
(43) Veröffentlichungstag der Anmeldung: 24.07.2002
(60) Teilanmeldung: 05019175.8
(73) Patentinhaber: Aesculap AG & Co. KG, 78532 Tuttlingen (DE)
(72) Erfinder: LERCH, Karl-Dieter, 58452 Witten (DE); FISCHER, Manfred, 78532 Tuttlingen (DE); STEINHILPER, Klaus-Dieter, 78532 Tuttlingen (DE); WEISSHAUPT, Dieter, 78194 Immendingen (DE)
(74) Vertreter: Hoeger, Stellrecht & Partner Patentanwälte
(86) Internationale Anmeldenummer: PCT/EP2000/010547
(87) Internationale Veröffentlichungsnummer: WO 2001/032089

(56) Entgegenhaltungen:
- EP-A- 0 787 466
- WO-A-00/49949
- DE-C- 19 832 797
- US-A- 5 921 986
- PATENT ABSTRACTS OF JAPAN vol. 017, no. 670 (C-1139), 9. Dezember 1993 (1993-12-09) -& JP 05 220174 A (Y. SHIMA), 31. August 1993 (1993-08-31)

## Beschreibung

Die Erfindung betrifft ein chirurgisches Verbindungselement zur Fixierung benachbart angeordneter Knochenplatten, umfassend ein erstes Anlageelement zur Anlage an die Knochenplatten, ein zweites Anlageelement zur Anlage an die Knochenplatten und ein Kopplungselement, welches durch einen Zwischenraum zwischen den Knochenplatten führbar ist und mittels welchem erstes und zweites Anlageelement derart miteinander koppelbar sind, daß zwischen dem ersten und zweiten Anlageelement liegende Knochenplatten fixierbar sind, wobei das zweite Anlageelement und das Kopplungselement so ausgebildet und aufeinander abgestimmt sind, daß durch Relativbewegung zwischen dem Kopplungselement und dem zweiten Anlageelement quer zu einer Klemmrichtung die Knochenplatten zwischen dem ersten und dem zweiten Anlageelement fixierbar sind.

Um dem Operateur einen Zugang zum Operationsbereich zu eröffnen, wird beispielsweise bei craniochirurgischen Eingriffen die Schädelkapsel des Patienten dadurch geöffnet, daß mittels Craniotomschnitten unter Ausbildung eines Schnittspalts ein Kalottensegment der Schädelkapsel entnommen wird, um so einen Zugang zum darunter liegenden Gehirn zu erhalten. Im Anschluß an die Operation muß das der Schädelkapsel entnommene Kalottensegment wieder in die Schädelkapsel eingefügt und an der verbliebenen Schädelkalotte fixiert werden.

Aus der DE 296 14 921 U1 ist ein chirurgisches Verbindungselement zur Fixierung eines einer Schädelkapsel entnommenen Kalottensegments an der verbliebenen Schädelkalotte bekannt, bei welchem das Verbindungselement einen in einen Schnittspalt zwischen Kalottensegment und verbliebener Schädelkalotte einführbaren Verbindungsschaft, einen Verbindungskopf zum Überdecken des Schnittspaltes sowie ein vom Verbindungsschaft seitlich vorspringendes, am Kalottensegment sowie der verbliebenen Schädelkalotte anlegbares Anlageelement umfaßt.

Aus der EP 0 787 466 ist eine Vorrichtung zum Fixieren eines aus der Schädelkapsel zum Zwecke eines operativen Eingriffs herausgetrennten Knochenstücks in einem am verbliebenen Schädelbein freigelegten Bereich bekannt, wobei eine mittig gelochte Scheibe als erstes und zweites Anlageelement auf einen Schaft als Kopplungselement aufschiebbar sind und eine Scheibe ausgehende Radialschnitte aufweist. Zum Fixieren der Scheibe an dem Schaft wird ein Werkzeug verwendet. Auch eine Gewindeverbindung kann zum Einsatz kommen.

Aus der US 4,802,477 ist eine Vorrichtung zum Sichern eines Sternums in einer geschlossenen Stellung nach Öffnung bekannt. Ein entsprechender Fixierungskragen greift in eine Gewindestange ein.

Aus der JP 5-220174 ist eine Fixierungsvorrichtung für Knochenplatten bekannt, bei welcher ein erstes Anlageelement mittels einer sägezahnförmigen Zahnleiste und einem zylindrischen Teil verklemmt werden.

Die US 5,921,986 offenbart ein Verfahren zur Behandlung eines Knochenbruchs, wobei ein erstes Anlageelement an ein erstes Knochenteil angelegt wird und ein zweites Anlageelement an ein zweites Knochenteil. Die beiden Knochenteile werden über die Anlageelement miteinander verspannt. Dazu ist ein Faden vorgesehen, welcher durch eine zuvor gebohrte Passage geführt wird.

Es ist Aufgabe der Erfindung, ein gattungsgemäßes chirurgisches Verbindungselement derart auszubilden, daß es einfach und auf sichere Weise einsetzbar und auf kostengünstige Weise herstellbar ist und insbesondere körperverträgliche und resorbierbare Materialien verwendbar sind.

Diese Aufgabe wird bei einem chirurgischen Verbindungselement der eingangs beschriebenen Art erfindungsgemäß dadurch gelöst, daß das Kopplungselement ein linear flexibler Faden oder Draht ist und daß das erste und/oder zweite Anlageelement einen Haltekörper aufweisen, welcher in den Zwischenraum zwischen den Knochenplatten einsetzbar ist.

Durch eine derartige Ausgestaltung muß keine Gewindeverbindung oder Klemmverbindung über den ganzen Umfang eines Schafts mehr vorgesehen werden, sondern die Kopplung zwischen dem zweiten Anlageelement und dem Kopplungselement läßt sich auf einfache und doch sichere Weise ausbilden. Dadurch hat man auch eine größere Auswahl an Materialien, die zur Herstellung des chirurgischen Verbindungselements einsetzbar sind, da kein Gewinde mehr geschnitten werden muß.

Bei einem Gewindeschnitt oder auch bei einer Verklemmung über den ganzen Umfang sind bevorzugterweise Metalle zu verwenden, um eine gute Fixierung zu erhalten. Durch die erfindungsgemäße Kopplung, die davon abgeht, insbesondere einen Gewindeschnitt vorsehen zu müssen, lassen sich dann auch beispielsweise weichere Kunststoffmaterialien einsetzen, die eine hohe Körperverträglichkeit oder Resorptionsfähigkeit aufweisen und mit denen sich durch den erfindungsgemäßen Fixierungs- und Kopplungsmechanismus sich eine gute und sichere Verbindung herstellen läßt.

Durch die erfindungsgemäße Ausgestaltung läßt sich insbesondere eine kraftschlüssige Verbindung zwischen dem zweiten Anlageelement und dem Kopplungselement herstellen.

Das Kopplungselement ist linear flexibel ausgebildet. Dies bedeutet, daß bezogen auf ein Linienelement das Kopplungselement radial-isotrop flexibel ist. Das Kopplungselement ist durch einen Faden oder Draht gebildet. Durch ein derartig ausgebildetes Kopplungselement läßt sich insbesondere ein Abstand zwischen dem ersten und zweiten Anlageelement stufenlos einstellen.

Um die Verklemmung von zwischen dem ersten und dem zweiten Anlageelement eingeklemmten Knochenplatten zu verbessern, ist es vorgesehen, daß das erste und/oder zweite Anlageelement einen Haltekörper aufweisen, welcher in einen Zwischenraum zwischen benachbart angeordneten Knochenplatten einsetzbar ist. Ein solcher Haltekörper dient als zusätzliche Verkeilung und insbesondere bewirkt er auch eine zusätzliche Fixierung in einer Richtung quer zur Klemmrichtung.

Besonders vorteilhaft ist es, wenn eine Fixierungsstellung des ersten und des zweiten Anlageelements durch Relativbewegung zwischen dem Kopplungselement und dem zweiten Anlageelement quer zur Klemmrichtung verriegelbar ist. Dadurch läßt sich auf einfache Weise mit kostengünstig herstellbaren Verbindungselementen eine sichere Fixierung erreichen.

Aus demselben Grund ist es besonders günstig, wenn eine Fixierungsstellung des ersten und des zweiten Anlageelements durch Relativbewegung zwischen dem Kopplungselement und dem zweiten Anlageelement quer zur Klemmrichtung entriegelbar ist. Beispielsweise läßt sich dann eine Nachjustierung auf einfache Weise während einer Operation durchführen.

Ganz besonders vorteilhaft ist es, wenn ein Haltekörper in Querrichtung zur Klemmrichtung elastisch ausgebildet ist und insbesondere federnd ausgebildet ist. Dadurch läßt sich in dieser Querrichtung der Abstand der Knochenplatten, welche an dem Haltekörper anliegen, in bestimmten Grenzen variieren und insbesondere läßt sich bei der Verwendung von mehreren Verbindungselementen eine Verspannung einer Knochenplatte gegenüber umgebenden Knochenplatten in Querrichtung.erreichen.

Da die Verbindungselemente im Körper verbleiben, ist es vorteilhaft, wenn ein solches Verbindungselement aus einem körperverträglichen Material hergestellt ist. Ganz besonders vorteilhaft ist es, wenn das Verbindungselement aus einem resorbierbaren Kunststoffmaterial gefertigt ist. Schnittspalte zwischen den Knochenplatten wachsen nach der Operation wieder zu und bei resorbierbarem Material kann Gewebe auch über das Verbindungselement wachsen, so daß dieses kein "Störfaktor" im Körper ist.

Kostengünstig ist es, wenn das erste und das zweite Anlageelement im wesentlichen gleich ausgebildet sind, da dadurch die Herstellung vereinfacht ist.

Günstigerweise ist dabei das erste Anlageelement auf funktional gleiche Weise wie das zweite Anlageelement mit dem Kopplungselement koppelbar.

Günstig ist es, wenn das erste Anlageelement in einem Abstand angeordnete Ausnehmungen zum Durchfädeln des linear flexiblen Kopplungselements aufweist. Zwischen den beiden Ausnehmungen läßt sich dann mit jeweils durchgezogenen Fadenenden eine Schleife bilden, und durch Zug an den beiden Fadenenden läßt sich über die Schleife das erste Anlageelement unter Zugspannung setzen, und so beispielsweise zu einer Knochenplatte hin ziehen.

Weiterhin ist es vorteilhaft, wenn das zweite Anlageelement mindestens eine Ausnehmung zum Durchführen des flexiblen Kopplungselements aufweist. Dadurch wird die Fixierung, die eine Fixierung des ersten und des zweiten Anlageelements relativ zueinander umfaßt, erleichtert.

Bei einer ersten Variante einer Ausführungsform sind das erste und das zweite Anlageelement mit dazwischenliegenden Knochenplatten durch einen Knoten im linear flexiblen Kopplungselement fixierbar. Bei entsprechender Wahl des Knotens läßt sich eine Zugspannung ausüben, die das erste und das zweite Anlageelement aufeinanderzubewegt und dazwischenliegende Knochenplatten gegeneinander fixiert. Der Knoten selber fixiert dann diese Fixierungsstellung.

Bei einer zweiten Variante einer Ausführungsform ist es vorgesehen, daß das zweite Anlageelement ein Pflockelement zum Fixieren des linear flexiblen Kopplungselements durch Umschleifen aufweist. Ein linear flexibles Kopplungselement, beispielsweise ein Faden oder ein Draht, läßt sich auf einfache Weise um das pflockelement schlingen oder wickeln und wird so im wesentlichen durch Reibungskraft an dem Pflockelement gehalten. Dadurch läßt sich ein Abstand zwischen den beiden Anlageelementen fixieren. Bei entsprechender Wahl der Umwicklungsschleifen läßt sich noch eine Zugspannung ausüben, die das erste und das zweite Anlageelement aufeinander zu bewegt und durch weitere Umwicklungen läßt sich dann eine Fixierungsstellung sichern. Zusätzlich kann dann noch nach erfolgter Umwicklung ein Knoten vorgesehen werden.

Günstigerweise ist dabei das Pflockelement vertieft angeordnet, so daß es nicht oder nur wenig über eine äußere Oberfläche des zweiten Anlageelements hinausragt, d. h. dessen äußere Abmessungen im wesentlichen nicht vergrößert.

Ganz besonders vorteilhaft ist es, wenn eine Fixierungsausnehmung des zweiten Anlageelements eine Klemmaufnahme für das linear flexible Kopplungselement umfaßt. Durch Einführung beispielsweise eines Fadens in diese Klemmaufnahme ist dieser gesichert, d. h. gegenüber dem zweiten Anlageelement fixiert. Auf diese Weise läßt sich dann ein Abstand zwischen dem ersten und dem zweiten Anlageelement fixieren und damit lassen sich auch nebeneinanderliegende Knochenplatten gegeneinander fixieren.

Günstigerweise ist dabei eine Klemmaufnahme in einem radialen Abstand bezogen auf eine Projektion einer Durchführungsöffnung des ersten Anlageelements auf das zweite Anlageelement angeordnet. Dadurch läßt sich eine verbesserte Klemmwirkung erzielen, da beim Einbringen des linear flexiblen Kopplungselements in eine Klemmaufnahme durch Bewegung quer zur Klemmrichtung diese nicht verringert wird.

Ein erfindungsgemäßes Verbindungselement läßt sich auf einfache Weise einsetzen und kostengünstig herstellen, wenn das zweite Anlageelement kraftschlüssig an dem Kopplungselement fixierbar ist.

Ganz besonders vorteilhaft ist es, wenn die Relativbewegung zwischen dem zweiten Anlageelement und dem. Kopplungselement zur Fixierung des zweiten Anlageelements mit dem Kopplungselement ausgehend von einer nicht fixierten Stellung in eine Fixierungsstellung erfolgt, denn dadurch läßt sich auf einfache Weise eine Verriegelung herstellen oder wieder aufheben.

Die nachfolgende Beschreibung bevorzugter Ausführungsformen der Erfindung dient im Zusammenhang mit der Zeichnung der näheren Erläuterung. Es zeigen:
- Fig. 1: eine perspektivische Ansicht eines einer Schädelkapsel entnommenen Kalottensegments, welches wieder in die verbliebene Schädelkalotte eingesetzt ist, wobei nebeneinander liegende Knochenplatten mit Hilfe von Verbindungselementen gemäß einer ersten Ausführungsform fixiert sind;
- Fig. 2: eine perspektivische Ansicht einer zweiten Ausführungsform eines erfindungsgemäßen Verbindungselements mit fixierten Knochenplatten; und
- Fig. 3: eine perspektivische Ansicht einer dritten Ausführungsform eines Verbindungselements mit fixierten Knochenplatten.

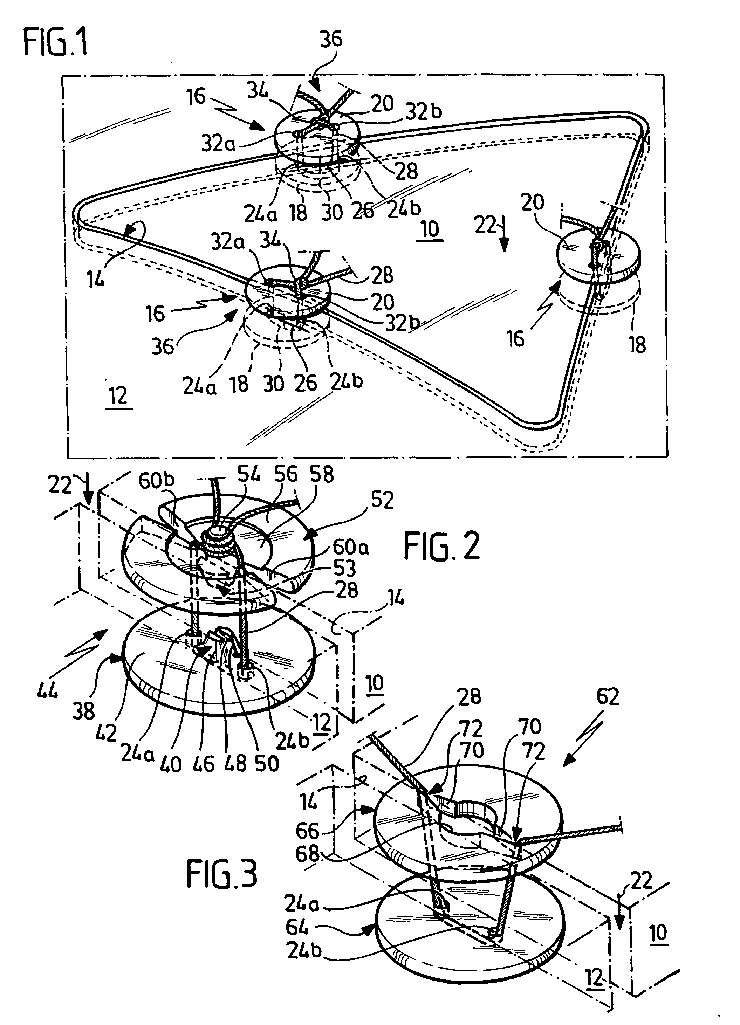

Ein Anwendungsbeispiel für ein Verbindungselement ist die Fixierung eines Kalottensegments 10, wie in Fig. 1 gezeigt, an einer umgebenden verbliebenen Schädelkalotte 12. Das Kalottensegment 10 wurde durch Craniotomschnitte entfernt, welche einen Schnittspalt 14 gebildet haben, um einem Operateur einen Zugang zum unter dem Kalottensegment 10 liegenden Gehirn zu verschaffen. Nach erfolgter Operation wird dieses Kalottensegment 10 wieder eingesetzt. Mit Hilfe der Verbindungselemente 16, beispielsweise mittels dreier solcher Verbindungselemente 16, wie in Fig. 1 dargestellt, werden die benachbarten Knochenplatten 10 und 12 fixiert, so daß das Kalottensegment 10 wieder mit der Schädelkalotte 12 verwachsen kann. Dazu ist ein solches Verbindungselement 16 aus einem körperverträglichen Material gefertigt und insbesondere aus einem resorbierbaren Material, so daß Körpergewebe mit dem Verbindungselement verwachsen kann.

Bei einer ersten Ausführungsform eines Verbindungselements, welche in Fig. 1 gezeigt ist, umfaßt ein solches Verbindungselement 16 ein erstes scheibenförmiges Anlageelement 18 und ein zweites Anlageelement 20, welches grundsätzlich gleich aufgebaut ist wie das erste Anlageelement. Das erste Anlageelement 18 wird unter das Kalottensegment 10 und die Schädelkalotte 12 gelegt und das zweite Anlageelement 20 in einem Abstand dazu über das Kalottensegment 10 und die Schädelkalotte 12. Durch Fixierung des ersten Anlageelements 18 relativ zum zweiten Anlageelement 20 und Ausübung einer Klemmkraft in eine Klemmrichtung 22, welche bei dem gezeigten Ausführungsbeispiel im wesentlichen senkrecht zu den Knochenplatten 10 und 12 ist, lassen sich dann das benachbarte Kalottensegment 10 und die Schädelkalotte 12 gegeneinander fixieren.

Das erste Anlageelement 18 weist zwei in einem Abstand angeordnete Durchführungsausnehmungen 24a, 24b auf, deren Verbindungslinie 26 bevorzugterweise auf einer Durchmesserlinie liegt. Ein Faden oder Draht 28 als Kopplungselement, welches linear flexibel ist in dem Sinne, daß es bezogen auf einen tangentialen Richtungsvektor senkrecht zu diesem Richtungsvektor keine Vorzugsrichtung bezüglich der Flexibilität gibt, ist so durch die beabstandeten Durchführungsausnehmungen 24a und 24b im ersten Anlageelement 18 geschleift, daß zwischen den beiden Durchführungsausnehmungen 24a und 24b ins Schädelinnere weisend eine Schleife 30 gebildet ist.

Das Kopplungselement 28 ist dann durch den Schnittspalt 14 vom Schädelinneren weg durch entsprechende Ausnehmungen 32a, b im zweiten Anlageelement 20 geführt. Wird eine Zugkraft nach außen (entgegen der Klemmrichtung 22) auf entsprechende Enden des Kopplungselements 28 ausgeübt, dann bewirkt dies, daß die Schleife 30 an dem ersten Anlageelement 18 anliegt.

Bei dem in Fig. 1 gezeigten Ausführungsbeispiel sind das erste Anlageelement 18 und das zweite Anlageelement 20 über einen Knoten 34 gegeneinander fixiert. Dazu werden entsprechende Enden des Kopplungselements 28 nach Durchschleifen durch die Ausnehmungen 32a und 32b des zweiten Anlageelements und Anziehen des Kopplungselements quer zur Klemmrichtung 22 geführt und miteinander verknotet, wobei eine Zugspannung ausgeübt wird, um eine Klemmkraft dadurch auszuüben, daß über die Schleife 30 das zweite Anlageelement 20 nach oben gezogen wird und gegen das Kalottensegment 10 und die Schädelkalotte 12 gespannt ist. Durch den Knoten 34 wird diese Fixierungsstellung oder Klemmstellung gesichert, d. h. die Fixierungsstellung 36 wird verriegelt.

Auf diese Weise sind dann über die Klemmwirkung des ersten Anlageelements 18 und des zweiten Anlageelements 20 auf die zwischenliegenden Knochenplatten das Kalottensegment 10 und die Schädelkalotte 12 zumindest in dem Bereich um ein Verbindungselement relativ zueinander fixiert. Durch die Verwendung mehrerer solcher Verbindungselemente 16 läßt sich dann das Kalottensegment 10 gegenüber der verbliebenen Schädelkalotte 12 im gesamten fixieren.

Bei einem weiteren Ausführungsbeispiel, welches in Fig. 2 gezeigt ist, ist das erste Anlageelement 38 grundsätzlich gleich aufgebaut, wie für das erste Ausführungsbeispiel beschrieben. Jedoch weist das erste Anlageelement bevorzugterweise zentral angeordnet einen Haltekörper 40 auf, welcher sich über eine Anlagefläche 42 erhebt. Das erste Anlageelement 38 wird bei dieser zweiten Ausführungsform 44 eines Verbindungselements mit dem Haltekörper 40 in den Schnittspalt 14 zwischen den benachbart angeordneten Knochenplatten 10 und 12 positioniert, um eine zusätzliche Verkeilung und damit eine bessere Fixierung zwischen den beiden Knochenplatten 10 und 12 zu bewirken.

Bevorzugterweise ist der Haltekörper 40 quer zur Klemmrichtung 22 elastisch und insbesondere federnd ausgebildet, so daß eine gewisse Variabilität bei der Bewegung der beiden Knochenplatten 10 und 12 aufeinander zu oder voneinander weg besteht, um ein optimales Fixierungsergebnis zu erreichen.

Bei dem in Fig. 2 gezeigten Ausführungsbeispiel umfaßt der Haltekörper 40 zwei gegenüberliegende Stiftteile 46 und 48, zwischen welchen ein Zwischenraum 50 gebildet ist. Die Stiftteile 46 und 48 sind dabei nach außen kegelstumpfförmig ausgebildet. Der Zwischenraum 50 bestimmt im wesentlichen, wie weit die Knochenplatten im Schnittspalt aufeinanderzuschiebbar sind; stoßen die beiden Stiftteile 46 und 48 aneinander, dann ist der minimale Abstand zwischen den Knochenplatten erreicht.

Bei der zweiten Ausführungsform 44 ist im Zentrum des zweiten Anlageelements 52 ein Pflockelement 54 angeordnet, welches insbesondere in einer gegenüber einer äußeren Oberfläche 56 gebildeten Vertiefung 58 sitzt, damit dieses Pflockelement 54 nicht oder nur geringfügig über die Oberfläche 56 hinausragt.

In dem zweiten Anlageelement 52 sind in radialer Richtung seitlich offene keilförmige Ausnehmungen 60a, 60b gebildet, durch die das flexible Kopplungselement 28 einführbar ist, um es um das Pflockelement 24 umschleifen und insbesondere umwickeln zu können.

Die Klemmkraft zwischen dem ersten Anlageelement 38 und dem zweiten Anlageelement 52 wird grundsätzlich, wie bereits oben für die erste Ausführungsform beschrieben, erzeugt. Die Fixierungsstellung wird dabei dadurch verriegelt, d. h. fixiert, daß die jeweiligen Enden des linear flexiblen Kopplungselements 28 um das Pflockelement 54 so gewickelt werden, daß die beiden Lageelemente 38 und 52 gegeneinander mit dazwischenliegenden Knochenplatten 10 und 12 gehalten sind. Die Ausnehmungen 60a und 60b dienen dabei als Einführungsschlitze für ein fadenförmiges Kopplungselement 28.

Das zweite Anlageelement 52 kann dabei ebenfalls einen zentral angeordneten Haltekörper 53 aufweisen, der insbesondere gleich wie der Haltekörper 40 des ersten Anlageelements ausgebildet ist.

Bei einer dritten Ausführungsform eines Verbindungselements 62, welche in Fig. 3 gezeigt ist, ist das erste Anlageelement 64 wie das erste Anlageelement 18 gemäß dem erstem Ausführungsbeispiel (Fig. 1) ausgebildet. Das zweite Anlageelement 66 weist eine zentrale Öffnung 68 als Durchführungsausnehmung für das linear flexible Kopplungselement 28 zum Durchführen von diesem auf. Ausgehend von dieser zentralen Öffnung 68 sind gegenüberliegend in radialer Richtung keilförmige Ausnehmungen 70 gebildet. Am radial außen liegenden Ende einer solchen Ausnehmung 70 ist eine Klemmaufnahme 72 als Fixierungsausnehmung gebildet, in die das flexible Kopplungselement 28 einklemmbar ist, um dieses gegenüber dem zweiten Anlageelement 66 zu fixieren.

Eine Klemmaufnahme 72 ist dabei bevorzugterweise bezogen auf eine Projektion einer Durchführungsausnehmung 24a bzw. 24b des ersten Anlageelements 64 in Klemmrichtung 22 auf das zweite Anlageelement 66 (bei entsprechender Ausrichtung von erstem Anlageelement 64 und zweitem Anlageelement 66) in radialer Richtung weiter außen liegend. Dadurch lassen sich durch Zug quer zur Klemmrichtung 22 Knochenplatten zwischen dem ersten Anlageelement 64 und dem zweiten Anlageelement einklemmen, wobei mittels der Klemmaufnahme 72 eine Fixierungsstellung fixierbar ist.

## Patentansprüche

1. Chirurgisches Verbindungselement zur Fixierung benachbart angeordneter Knochenplatten (10, 12), umfassend ein erstes Anlageelement (38) zur Anlage an die Knochenplatten (10, 12), ein zweites Anlageelement (20, 52, 66) zur Anlage an die Knochenplatten (10, 12) und ein Kopplungselement (28), welches durch einen Zwischenraum (14) zwischen den Knochenplatten (10, 12) führbar ist und mittels welchem erstes und zweites Anlageelement derart miteinander koppelbar sind, daß zwischen dem ersten und zweiten Anlageelement liegende Knochenplatten (10, 12) fixierbar sind, wobei das zweite Anlageelement (20, 52, 66) und das Kopplungselement (28) so ausgebildet und aufeinander abgestimmt sind, daß durch Relativbewegung zwischen dem Kopplungselement (28) und dem zweiten Anlageelement (20, 52, 66) quer zu einer Klemmrichtung (22) die Knochenplatten (10, 12) zwischen dem ersten und dem zweiten Anlageelement fixierbar sind,
**dadurch gekennzeichnet, daß** das Kopplungselement (28) ein linear flexibler Faden oder Draht ist und daß das erste und/oder zweite Anlageelement (38; 52) einen Haltekörper (40; 53) aufweisen, welcher in den Zwischenraum (14) zwischen den Knochenplatten (10, 12) einsetzbar ist.

2. Chirurgisches Verbindungselement nach Anspruch 1, **dadurch gekennzeichnet, daß** eine Fixierungsstellung des ersten und des zweiten Anlageelements durch Relativbewegung zwischen dem Kopplungselement und dem zweiten Anlageelement (20, 52, 66) quer zur Klemmrichtung verriegelbar ist.

3. Chirurgisches Verbindungselement nach Anspruch 1 oder 2, **dadurch gekennzeichnet, daß** eine Fixierungsstellung des ersten und des zweiten Anlageelements durch Relativbewegung zwischen dem Kopplungselement (28) und dem zweiten Anlageelement (20, 52, 66) quer zur Klemmrichtung entriegelbar ist.

4. Chirurgisches Verbindungselement nach einem der vorangehenden Ansprüche, **dadurch gekennzeichnet, daß** ein Haltekörper (53) in Querrichtung zur Klemmrichtung (22) elastisch ausgebildet ist.

5. Chirurgisches Verbindungselement nach einem der vorangehenden Ansprüche, **dadurch gekennzeichnet, daß** das Verbindungselement aus einem körperverträglichen Material hergestellt ist.

6. Chirurgisches Verbindungselement nach einem der vorangehenden Ansprüche, **dadurch gekennzeichnet, daß** das Verbindungselement aus einem resorbierbaren Kunststoffmaterial gefertigt ist.

7. Chirurgisches Verbindungselement nach einem der vorangehenden Ansprüche, **dadurch gekennzeichnet, daß** das erste (38) und das zweite (52) Anlageelement im wesentlichen gleich ausgebildet sind.

8. Chirurgisches Verbindungselement nach einem der vorangehenden Ansprüche, **dadurch gekennzeichnet, daß** zwischen dem ersten Anlageelement (38) und dem zweiten Anlageelement (20; 52; 66) sitzende Knochenplatten durch eine Relativbewegung des Kopplungselements (28) zum zweiten Anlageelement (20; 52; 66) fixierbar sind.

9. Chirurgisches Verbindungselement nach einem der vorangehenden An-sprüche, **dadurch gekennzeichnet, daß** das erste Anlageelement (38) in einem Abstand angeordnete Ausnehmungen (24a, 24b) zum Durchfädeln des linear flexiblen Kopplungselements (28) aufweist.

10. Chirurgisches Verbindungselement nach einem der vorangehenden Ansprüche, **dadurch gekennzeichnet, daß** das zweite Anlageelement (20) mindestens eine Ausnehmung (32a; 32b) zum Durchführen des flexiblen Kopplungselements (28) aufweist.

11. Chirurgisches Verbindungselement nach einem der vorangehenden Ansprüche, **dadurch gekennzeichnet, daß** das erste (38) und das zweite (20) Anlageelement mit dazwischenliegenden Knochenplatten durch einen Knoten (34) im linear flexiblen Kopplungselement (28) fixierbar sind.

12. Chirurgisches Verbindungselement nach einem der vorangehenden Ansprüche, **dadurch gekennzeichnet, daß** das zweite Anlageelement (52) ein Pflockelement (54) zur Fixierung des linear flexiblen Kopplungselements (28) durch Umschleifen aufweist.

13. Chirurgisches Verbindungselement nach Anspruch 12, **dadurch gekennzeichnet, daß** das Pflockelement (54) vertieft angeordnet ist.

14. Chirurgisches Verbindungselement nach einem der vorangehenden Ansprüche, **dadurch gekennzeichnet, daß** eine Fixierungsausnehmung des zweiten Anlageelements (66) eine Klemmaufnahme (72) für das linear flexible Kopplungselement (28) umfaßt.

15. Chirurgisches Verbindungselement nach Anspruch 14, **dadurch gekennzeichnet, daß** gegenüberliegende Klemmaufnahmen (72) vorgesehen sind.

16. Chirurgisches Verbindungselement nach Anspruch 14 oder 15, **dadurch gekennzeichnet, daß** eine Klemmaufnahme (72) in einem radialen Abstand bezogen auf eine Projektion einer Durchführungsöffnung (24a; 24b) des ersten Anlageelements (64) in Klemmrichtung (22) auf das zweite Anlageelement (66) angeordnet ist.

17. Chirurgisches Verbindungselement nach einem der vorgenannten Ansprüche, **dadurch gekennzeichnet, daß** das zweite Anlageelement kraftschlüssig an dem Kopplungselement fixierbar ist.

18. Chirurgisches Verbindungselement nach einem der vorgenannten Ansprüche, **dadurch gekennzeichnet, daß** die Relativbewegung zwischen dem zweiten Anlageelement und dem Kopplungselement zur Fixierung des zweiten Anlageelements mit dem Kopplungselement ausgehend von einer nicht fixierten Stellung in eine Fixierstellung erfolgt.

## Claims

1. A surgical connecting element for fixing adjacently arranged bone plates (10, 12), comprising a first bearing element (38) for bearing on the bone plates (10, 12), a second bearing element (20, 52, 66) for bearing on the bone plates (10, 12) and a coupling element (28), which can be guided through an intermediate space (14) between the bone plates (10, 12) and by means of which first and second bearing element can be coupled to each other such that bone plates (10, 12) lying between the first and second bearing element can be fixed, wherein the second bearing element (20, 52, 66) and the coupling element (28) are so designed and coordinated with each other that, by relative movement between the coupling element (28) and the second bearing element (20, 52, 66) transversely to a clamping direction (22), the bone plates (10, 12) can be fixed between the first and the second bearing element,
**characterised in that** the coupling element (28) is a linearly flexible thread or wire, and **in that** the first and/or second bearing element (38; 52) have a holding body (40; 53) which can be inserted into the intermediate space (14) between the bone plates (10, 12).

2. A surgical connecting element according to Claim 1, **characterised in that** a fixing position of the first and of the second bearing element can be locked by relative movement between the coupling element and the second bearing element (20, 52, 66) transversely to the clamping direction.

3. A surgical connecting element according to Claim 1 or 2, **characterised in that** a fixing position of the first and of the second bearing element can be unlocked by relative movement between the coupling element (28) and the second bearing element (20, 52, 66) transversely to the clamping direction.

4. A surgical connecting element according to one of the preceding claims, **characterised in that** a holding body (53) is designed to be elastic in the direction transverse to the clamping direction (22).

5. A surgical connecting element according to one of the preceding claims, **characterised in that** the connecting element is produced from a biocompatible material.

6. A surgical connecting element according to one of the preceding claims, **characterised in that** the connecting element is fabricated from a resorbable plastics material.

7. A surgical connecting element according to one of the preceding claims, **characterised in that** the first (38) and the second (52) bearing element are of substantially the same construction.

8. A surgical connecting element according to one of the preceding claims, **characterised in that** the bone plates seated between the first bearing element (38) and the second bearing element (20; 52; 66) can be fixed by relative movement of the coupling element (28) to the second bearing element(20; 52; 66).

9. A surgical connecting element according to one of the preceding claims, **characterised in that** the first bearing element (38) has spaced-apart openings (24a, 24b) for threading through the linearly flexible coupling element (28).

10. A surgical connecting element according to one of the preceding claims, **characterised in that** the second bearing element (20) has at least one opening (32a; 32b) for feeding through the flexible coupling element (28).

11. A surgical connecting element according to one of the preceding claims, **characterised in that** the first(38) and the second (20) bearing element can be fixed with bone plates lying therebetween by a knot (34) in the linearly flexible coupling element (28).

12. A surgical connecting element according to one of the preceding claims, **characterised in that** the second bearing element (52) has a peg element (54) for fixing the linearly flexible coupling element (28) by looping it around.

13. A surgical connecting element according to Claim 12, **characterised in that** the peg element (54) is arranged in a recess.

14. A surgical connecting element according to one of the preceding claims, **characterised in that** a fixing opening of the second bearing element (66) comprises a clamping seat (72) for the linearly flexible coupling element (28).

15. A surgical connecting element according to claim 14, **characterised in that** opposite clamping seats (72) are provided.

16. A surgical connecting element according to claim 14 or 15, **characterised in that** a clamping seat (72) is arranged at a radial spacing with respect to a projection of a feed-through opening (24a; 24b) of the first bearing element (64) in the clamping direction (22) onto the second bearing element (66).

17. A surgical connecting element according to one of the aforementioned claims, **characterised in that** the second bearing element can be fixed to the coupling element in force-transmitting manner.

18. A surgical connecting element according to one of the aforementioned claims, **characterised in that** the relative movement between the second bearing element and the coupling element for the fixing of the second bearing element to the coupling element proceeds from an unfixed position to a fixing position.

## Revendications

1. Élément de liaison chirurgical destiné à fixer des plaques osseuses (10, 12) adjacentes, comportant un premier élément d'appui (38) destiné à venir en appui sur les plaques osseuses (10, 12), un deuxième élément d'appui (20, 52, 66) destiné à venir en appui sur les plaques osseuses (10, 12) et un élément de couplage (28) susceptible d'être introduit par un intervalle (14) entre les plaques osseuses (10, 12) et au moyen duquel le premier et le deuxième élément d'appui peuvent être couplés l'un à l'autre de manière à pouvoir fixer des plaques osseuses (10, 12) se trouvant entre le premier et le deuxième élément d'appui, le deuxième élément d'appui (20, 52, 66) et l'élément de couplage (28) étant conçus et adaptés l'un à l'autre de telle sorte que, sous l'effet d'un mouvement relatif entre l'élément de couplage (28) et le deuxième élément d'appui (20, 52, 66) perpendiculairement à une direction de serrage (22), les plaques osseuses (10, 12) peuvent être fixées entre le premier et le deuxième élément d'appui,
**caractérisé en ce que** l'élément de couplage (28) est un fil non métallique ou métallique linéairement flexible et **en ce que** le premier et/ou le deuxième élément d'appui (38 ; 52) comportent un corps de retenue (40 ; 53) qui peut être placé dans l'intervalle (14) entre les plaques osseuses (10, 12).

2. Élément de liaison chirurgical selon la revendication 1, **caractérisé en ce qu'**une position de fixation du premier et du deuxième élément d'appui peut être verrouillée par un mouvement relatif entre l'élément de couplage (28) et le deuxième élément d'appui (20, 52, 66) perpendiculairement à la direction de serrage.

3. Élément de liaison chirurgical selon la revendication 1 ou 2, **caractérisé en ce qu'**une position de fixation du premier et du deuxième élément d'appui peut être déverrouillée par un mouvement relatif entre l'élément de couplage (28) et le deuxième élément d'appui (20, 52, 66) perpendiculairement à la direction de serrage.

4. Élément de liaison chirurgical selon l'une quelconque des revendications précédentes, **caractérisé en ce qu'**un corps de retenue (53) est conçu de manière à être élastique dans la direction perpendiculaire à la direction de serrage (22).

5. Élément de liaison chirurgical selon l'une quelconque des revendications précédentes, **caractérisé en ce que** l'élément de liaison est fabriqué en un matériau biocompatible.

6. Élément de liaison chirurgical selon l'une quelconque des revendications précédentes, **caractérisé en ce que** l'élément de liaison est réalisé en une matière plastique résorbable.

7. Élément de liaison chirurgical selon l'une quelconque des revendications précédentes, **caractérisé en ce que** le premier élément d'appui (38) et le deuxième élément d'appui (52) sont sensiblement identiques.

8. Élément de liaison chirurgical selon l'une quelconque des revendications précédentes, **caractérisé en ce que** des plaques osseuses, se trouvant entre le premier élément d'appui (38) et le deuxième élément d'appui (20 ; 52 ; 66), peuvent être fixées par un mouvement relatif de l'élément de couplage (28) par rapport au deuxième élément d'appui (20 ; 52 ; 66).

9. Élément de liaison chirurgical selon l'une quelconque des revendications précédentes, **caractérisé en ce que** le premier élément d'appui (38) comporte des évidements (24a, 24b) distants l'un de l'autre, destinés au passage de l'élément de couplage (28) linéairement flexible.

10. Élément de liaison chirurgical selon l'une quelconque des revendications précédentes, **caractérisé en ce que** le deuxième élément d'appui (20) comporte au moins un évidement (32a ; 32b) destiné au passage de l'élément de couplage (28) flexible.

11. Élément de liaison chirurgical selon l'une quelconque des revendications précédentes, **caractérisé en ce que** le premier élément d'appui (38) et le deuxième élément d'appui (20) peuvent être fixés, alors que sont intercalées des plaques osseuses, au moyen d'un noeud (34) dans l'élément de couplage (28) linéairement flexible.

12. Élément de liaison chirurgical selon l'une quelconque des revendications précédentes, **caractérisé en ce que** le deuxième élément d'appui (52) comporte une goupille (54) pour fixer par enroulement l'élément de couplage (28) linéairement flexible.

13. Élément de liaison chirurgical selon la revendication 12, **caractérisé en ce que** la goupille (54) est disposée en retrait.

14. Élément de liaison chirurgical selon l'une quelconque des revendications précédentes, **caractérisé en ce qu'**un évidement de fixation du deuxième élément d'appui (66) comporte un logement de serrage (72) pour l'élément de couplage (28) linéairement flexible.

15. Élément de liaison chirurgical selon la revendication 14, **caractérisé en ce qu'**il est prévu des logements de serrage (72) disposés face à face.

16. Élément de liaison chirurgical selon la revendication 14 ou 15, **caractérisé en ce qu'**un logement de serrage (72) est disposé à une distance radiale par rapport à une projection d'un orifice de passage (24a ; 24b) du premier élément d'appui (64), sur le deuxième élément d'appui (66), dans la direction de serrage (22).

17. Élément de liaison chirurgical selon l'une quelconque des revendications précédentes, **caractérisé en ce que** le deuxième élément d'appui peut être fixé par couplage de force sur l'élément de couplage.

18. Élément de liaison chirurgical selon l'une quelconque des revendications précédentes, **caractérisé en ce que** le mouvement relatif entre le deuxième élément d'appui et l'élément de couplage en vue de fixer le deuxième élément d'appui avec l'élément de couplage s'effectue à partir d'une position non fixée vers une position de fixation.
